# EUROPEAN PATENT APPLICATION

(11) **EP 2 546 222 A2**
(43) Date of publication of application: **16.01.2013**
(21) Application number: 12178204.9
(22) Date of filing: 03.01.2007
(51) Int. Cl.: C07C 17/087, C07C 21/19, C09K 5/00

(54) **Method for producing fluorinated organic compounds**

(30) Priority: 03.01.2006 US 755485 P
(62) Divisional of application: 07716229.5
(71) Applicant: Honeywell International Inc., Morristown, NJ 07960 (US)
(72) Inventor: Merkel, Daniel, C., West Seneca, NY 14224 (US); Tung, Hsueh, Sung, Getzville, NY 14068 (US); Van der Puy, Michael, Amherst, NY 14221 (US); Ma, Jing, Ji, West Seneca, NY 14224 (US); Dubey, Rajesh, Buffalo, NY 14210 (US); Light, Barbara, Niagra Falls, NY 14305 (US); Bortz, Cheryl, N Tonawanda, NY 14120 (US); Phillips, Steven, D., Buffalo, NY 14218 (US); Mukhopahyay, Sudip, Williamsville, NY 14221 (US)
(74) Representative: Crooks, Elizabeth Caroline

(57) **Abstract**

Disclosed are processes for the production of fluorinated olefins, preferably adapted to commercialization of CF₃CF=CH₂ (1234yf). Three steps may be used in preferred embodiments in which a feedstock such as CCl₂=CClCH₂Cl (which may be purchased or synthesized from 1,2,3- trichloropropane) is fluorinated (preferably with HF in gas-phase in the presence of a catalyst) to synthesize a compound such as CF₃CCl=CH₂, preferably in a 80-96% selectivity. The CF₃CCl=CH₂ is preferably converted to CF₃CFClCH₃ (244-isomer) using a SbCl₅ as the catalyst which is then transformed selectively to 1234yf, preferably in a gas-phase catalytic reaction using activated carbon as the catalyst. For the first step, a mixture of Cr₂O₃ and FeCl₃/C is preferably used as the catalyst to achieve high selectivity to CF₃CCl=CH₂ (96%). In the second step, SbCl₅/C is preferably used as the selective catalyst for transforming 1233xf to 244-isomer, CF₃CFClCH₃. The intermediates are preferably isolated and purified by distillation and used in the next step without further purification, preferably to a purity level of greater than about 95%.

## Description

### BACKGROUND OF INVENTION

### (1) Field ofInvention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkenes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions, Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts.

The preparation of HFO--1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

### SUMMARY

Applicants have discovered a method for producing fluorinated organic compounds, including hydrofluoropropenes, which preferably comprises converting at least one compound of Formula (I):

C(X)ₘCCl(Y)ₙC(X)ₘ (I)

to at least one compound of Formula (II)

CF₃CF=CHZ (II)

where each X, Y and Z is independently H, F, Cl, I or Br, and each m is independently 1, 2 or 3, and n is 0 or 1. As used herein and throughout, unless specifically indicated otherwise, the term "converting" includes directly converting (for example, in a single reaction or under essentially one set of reaction conditions, an example of which is described hereinafter) and indirectly converting (for example, through two or more reactions or using more than a single set of reaction conditions).

In certain preferred embodiments of the invention, the compound of Formula (1) comprises a compound wherein n is 0, each X is independently H or Cl, and Z is H. Such preferred embodiments include converting at least one C3 alkene in accordance with Formula (IA):

C(X)₂=CClC(X)₃ (IA)

to at least one compound of formula (II)

CF₃CF=CHZ (II)

where each X is independently H or Cl. Preferably the one or more compounds of Formula (IA) are tetrachloropropene(s), and are even more preferably selected from the group consisting of CH₂=CClCCl₃, CCl₂=CClCH₂Cl, CHCl=CClCCl₂H, and combinations of these.

In certain preferred embodiments of the invention the compound of Formula (I) comprises a compound wherein n is 0 and the terminal saturated carbon has three (3) F substituents. Such preferred embodiments include converting at least one C3 alkene in accordance with Formula (IAA):

C(X)₂=CClCF₃ (IAA)

to at least one compound of formula (II)

CF₃CF=CHZ (II)

where each X is independently H or Cl. Preferably the one or more compounds of Formula (IAA) are trifluoropropene(s). Included in the preferred trifluorpropene compounds of the present invention is CH₂=CClCF₃ (HCFC-1223xf).

In certain preferred embodiments the compound of Formula (I) comprises a compound wherein n is 1 and each X and Y is independently H, F or Cl. Such embodiments include converting at least one C3 alkane of Formula (IB):

C(X)₃CClYC(X)₃ (IB)

to at least one compound of formula (II)

CF₃CF=CHZ (II)

where each X and Y is independently H, F or Cl. In certain preferred embodiments, the Formula (IB) compound has at least two haologens on one terminal carbon and at least two hydrogen atoms on the other terminal carbon. Preferably the compounds of Formula (IB) contain at least four halogen substituents and even more preferably at least five halogen substituents. In certainly highly preferred embodiments, the conversion step of the present invention comprises converting a compound of Formula (IB) wherein Y is F and all three X on one terminal carbon are F. Preferably the compound of Formula (IB) is a penta-halogenated propane, preferably with at least four fluorine substituents. Even more preferably the penta-halogenated propane of Formula (IB) comprises a tetra-fluorinated, mono-chlorinated propane, including chlorotetrafluoropropane (C₃H₃F₄Cl), including all isomers thereof, such as 1,1,1,2-tetrafluoro-2-chloropropane and 1-chloro-1,3,3,3-tetrafluoropropane (HFC-244fa). Other preferred penta-halogenated compounds of Formula (IB) include CH₂CClCHClCCl₃, CHCl₂CCl₂CH₂Cl, CHCl₂CHClCHCl₂. Of course, combinations of compounds of Formula (I), including combinations of compounds of Formulas (IA), (IAA) and (IB) may be used.

In certain preferred embodiments, the step of converting a compound of Formula (I) to at least one compound of Formula (II) comprises directly converting a compound of Formula (I). In other embodiments, the step of converting a compound of Formula (I) to at least one compound of Formula (II) comprises indirectly converting a compound of Formula (I).

An example of indirect conversion embodiments includes converting a compound of Formula (IA) to a compound of Formula (IAA), then converting said Formula (IAA) compound to a Formula (IB) compound, and then converting the Formula (IB) to the Formula (II) compound. In certain more specific indirect conversion embodiments, the step of converting a compound of Formula (I) comprises providing at least one monchlortrifluorpropene in accordance with Formula (IAA), preferably CF₃CCl=CH₂ (HFO-1233xf) and reacting said monchlortrifluorpropene under conditions effective to produce at least one monchlortetrafluorpropane in accordance with Formula (IB), preferably CF₃CFClCH₃ (HFC-244bb), which in turn is preferably exposed to reaction conditions effective to produce at least one compound in accordance with Formula (II), preferably HFO-1234yf. In preferred embodiments said exposing step comprises conducting one or more of said reactions in a gas phase in the presence of a catalyst, preferably a metal-based catalyst. Examples of such preferred conversion steps are disclosed more fully hereinafter. Of course, it is contemplated that in the broad scope of the invention that any of the Formula (I) compounds may be converted, directly or indirectly, to a compound of Formula (II) in view of the teachings contained herein.

In certain preferred embodiments the converting step comprises exposing the compound of Formula (I), and preferably Formula (1A), (IAA) or Formula (1B), to one or more sets of reaction conditions effective to produce at least one compound in accordance with Formula (II). It is contemplated that in certain embodiments the exposing step comprises reacting said one or more compound(s) of Formula (IA) or (IAA) under conditions effective to produce chlorofluoropropane, more preferably a propane in accordance with Formula (IBB):

CF₃CClFC(X)₃ Formula (IBB)

where each X is independently F, Cl or H. In certain preferred embodiments, at least one of said X in Formula (IBB) is H, and even more preferably all three X are H.

The preferred conversion step of the present invention is preferably carried out under conditions, including the use of one or more reactions, effective to provide a Formula (I) conversion of at least about 50%, more preferably at least about 75%, and even more preferably at least about 90%. In certain preferred embodiments the conversion is at least about 95%, and more preferably at least about 97%. Further in certain preferred embodiments, the step of converting the compound of Formula (I) to produce a compound of Formula (II) is conducted under conditions effective to provide a Formula (II) yield of at least about 75%, more preferably at least about 85%, and more preferably at least about 90%. In certain preferred embodiments a yield of about 95% or greater is achieved.

### DETAILED DESCRIPTION OF PREFERRED EMBODIMENTS

One beneficial aspect of the present invention is that it enables the production of desirable fluroolefins, preferably C3 fluoroolefins, using relatively high conversion and high selectivity reactions. Furthermore, the present methods in certain preferred embodiments permit the production of the desirable fluoroolefins, either directly or indirectly, from relatively attractive starting materials. For example, 2-chloro, 2,3,3,3-tetrafluoropropane is a compound that may in certain embodiments be an advantageous starting material because such products are relatively easy to handle.

Preferably the Formula (I) compound is exposed to reaction conditions effective to produce a reaction product containing one or more of the desired fluorolefins, preferably one or more compounds of Formula (II). Although it is contemplated that the exposure step in certain embodiments may effectively be carried out in a single reaction stage and/or under a single set of reaction conditions, as mentioned above, it is preferred in many embodiments that the conversion stepcomprise a series of reaction stages or conditions. In one preferred aspect of the present invention, the conversion step comprises: (a) reacting a compound of Formula (I) which is not a compound of Formula (IAA), preferably a compound of Formula (IA), in a gas and/or liquid phase reaction in the presence of at least a first catalyst to produce at least one compound of Formula (IAA), such as a monochloro-trifluoro-propene, preferably HFO-1233xf; (b) reacting the at least one monochloro-trifluoro-propene compound, in a gas and/or liquid phase and preferably in the presence of at least a catalyst, preferably a second catalyst which is different than the first catalyst, to produce at least one compound of Formula (IB) and even more preferably Formula (IBB), such as monochloro-terafluoro-propane; and (c) reacting said compound of Formula (IB), in a gas and/or liquid phase, to produce the desired HFO, preferably HFO-1234yf. Each of the preferred reaction steps is described in detail below, with the headings being used for convenience but not necessarily by way of limitation.

### I. FLUORINATION OF THE COMPOUND OF FORMULA I(A)

One preferred reaction step in accordance with the present invention may be described by those reactions in which the compound of Formula (IA) is fluorinated to produce a compound of Formula (IAA). In certain preferred embodiments, especially embodiments in which the compound of Formula (IA) comprises C(X)₂=CClC(X)₃, where each X is independently H or Cl, the present converting step comprises first reacting said compound(s) by fluorinating said compound(s), preferably with HF in a gas phase, to produce an HFO that is at least trifluorinated, such as HFO-1223xf. Preferably this gas phase reaction is at least partially catalyzed.

The preferred fluorination of the compound of Formula (IA) is preferably carried out under conditions effective to provide a Formula (IA) conversion of at least about 50%, more preferably at least about 75%, and even more preferably at least about 90%. In certain preferred embodiments the conversion is at least about 95%, and more preferably at least about 97%. Further in certain preferred embodiments, the conversion of the compound of Formula (IA) comprises reacting such compound under conditions effective to produce at least one compound of Formula (IAA), such as monochlorotrifluoropropene (preferably CF₃CCl=CH₂ (HFO-1233xf)) at a selectivity of at least about 50%, more preferably at least about 70%, more preferably at least about 80%, and even more preferably at least about 90%, with selectivities of about 95% or greater being achieved in certain embodiments.

In general, it is possible that the fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of Formula (I) compounds, preferably Formula (IA) compounds, the reaction is at least partially a catalyzed reaction, and is preferably carried out on a continuous basis by introducing a stream containing the compound of Formula (I), preferably Formula (IA), into one or more reaction vessels, such as a tubular reactor. In certain preferred embodiments, the stream containing the compound of Formula (I), and preferably Formula (IA), is preheated to a temperature of from about80°C to about 400°C, more preferably from about 150°C to about 400°C, and in certain embodiments preferably about 300°C, and introduced into a reaction vessel (preferably a tube reactor), which is maintained at the desired temperature, preferably from about 80°C to about 700°C, more preferably from about 90°C to about 600°C , even more preferably in certain embodiments from about 400°C to about 600°C, more preferably from about 450°C to about 600°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained with the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a chromium-based catalyst (such as Cr₂O₃ catalyst), an iron-based catalyst (such as FeCl₃ on carbon (designated herein as FeCl₃/C for convenience), and combinations of these. In preferred embodiments, the catalyst is a combination of the two aforementioned catalysts, where the reaction vessel contains in a first zone the chromium-based catalyst and in a second zone the iron-based catalyst. The temperature of the reaction in the chromium-based catalyst reaction is preferably kept at a temperature of from about 200°C to about 600°C and even more preferably from about 250°C to about 500°C. The temperature of the reaction in the iron-based catalyst reaction zone is preferably kept at a temperature of from about 80°C to about 300°C and even more preferably from about 100°C to about 250°C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum, and in certain preferred embodiments is from about1 to about 200 psia, and in certain embodiments from about 1 to about 120 psia.

In certain embodiments, an inert diluent gas, such as nitrogen, may be used in combination with the other reactor feed(s).

It is contemplated that the amount of catalyst use will vary depending on the particular parameters present in each embodiment.

### II. FLUORINATION OF THE COMPOUND OF FORMULA I(AA)

The compound of Formula (IAA), preferably produced as described above, then is preferably subject to further fluorination reaction(s) to produce a compound of Formula (IB), such as HCFC-244. Preferably this gas phase reaction is at least partially catalyzed.

The fluorination of the compound of Formula (IAA) is preferably carried out under conditions effective to provide a Formula (IAA) conversion of at least about 40%, more preferably at least about 50%, and even more preferably at least about 60%. Further in certain preferred embodiments, the conversion of the compound of Formula (IA) comprises reacting such compound under conditions effective to produce at least one monochlorotetrafluoropropane, preferably HCFC-244, at a selectivity of at least about 70%, more preferably at least about 80%, and even more preferably at least about 85%, with selectivities of about 90% or greater being achieved in certain embodiments.

In general, it is possible that this fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of Formula (IAA) compounds, the reaction is at least partially a catalyzed reaction, and is preferably carried out on a continuous basis by introducing a stream containing the compound of Formula (IAA) into one or more reaction vessels, such as a tubular reactor. In certain preferred embodiments, the stream containing the compound of Formula (I), and preferably Formula (IAA), is preheated to a temperature of from about 50°C to about 400°C, and in certain embodiments preferably about 80°C. In other embodiments, it is preferred that the stream containing the compound of Formula (I), and preferably Formula (IAA), is preheated to a temperature of from about 150°C to about 400°C, preferably about 300°C. This steam, preferably after preheating, is then preferably introduced into a reaction vessel (preferably a tube reactor), which is maintained at the desired temperature, preferably from about 50°C to about 250°C, more preferably from about 50°C to about 150°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained within about the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably an Sb-based catalyst, such as catalyst which is about 50wt% SbCl₅/C. Other catalysts which may be used include: from about 3 to about 6 wt% FeCl₃/C; SbF₅/C; about 20 wt% SnCl₄/C; about 23 wt% TiCl₄/C; and activated carbon. Preferably the catalyst comprises Cl₂ and HF pre-treated SbCl₅/C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum and in certain preferred embodiments is from about 1 to about 200 psia, more preferably in certain embodiments from about 1 to about 120 psia.

In certain embodiments, an inert diluent gas, such as nitrogen, may be used in combination with the other reactor feed(s).

It is contemplated that the amount of catalyst use will vary depending on the particular parameters present in each embodiment.

### III. DEHYDROHALOGENATION OF FORMULA (IB)

One preferred reaction step in accordance with the present invention may be described by those reactions in which the compound of Formula (IB) is dehydrohalogenated to produce a compound of Formula (II) . In certain preferred embodiments, the stream containing the compound of Formula (IB), and preferably Formula (IBB) is preheated to a temperature of from about 150°C to about 400°C, preferably about 350°C, and introduced into a reaction vessel, which is maintained at about the desired temperature, preferably from about 200°C to about 700°C, more preferably from about 300°C to about 700°C, more preferably from about 300°C to about 450°C, and more preferably in certain embodiments from about 350°C to about 450°C.

Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

Thus, it is contemplated that the dehydrohalogenation reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a carbon- and/or metal-based catalyst, preferably activated carbon, a nickel-based catalyst (such as Ni-mesh) and combinations of these. Other catalysts and catalyst supports may be used, including palladium on carbon, palladium-based catalyst (including palladium on aluminum oxides), and it is expected that many other catalysts may be used depending on the requirements of particular embodiments in view of the teachings contained herein. Of course, two or more any of these catalysts, or other catalysts not named here, may be used in combination.

The gas phase dehydrohalogenation reaction may be conducted, for example, by introducing a gaseous form of a compound of Formula (IB) into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

While it is contemplated that a wide variety of reaction temperatures may be used, depending on relevant factors such as the catalyst being used and the most desired reaction product, it is generally preferred that the reaction temperature for the dehydrohalogentation step is from about 200°C to about 800°C, more preferably from about 400°C to about 800°C, and even more preferably from about 400°C to about 500°C, and more preferably in certain embodiments from about 300°C to about 500°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from about 1 to about 200 psia, and even more preferably in certain embodiments from about 1 to about 120 psia.

In certain embodiments, an inert diluent gas, such as nitrogen, may be used in combination with the other reactor feed(s), When such a diluent is used, it is generally preferred that the compound of Formula (I), preferably Formula (IB), comprise from about 50% to greater than 99% by weight based on the combined weight of diluent and Formula (I) compound.

It is contemplated that the amount of catalyst use will vary depending on the particular parameters present in each embodiment.

Preferably in such dehydrofluorination embodiments as described in this section, the conversion of the Formula (IB) compound is at least about 60%, more preferably at least about 75%, and even more preferably at least about 90%. Preferably in such embodiments, the selectivity to compound of Formula (II), preferably HFO-1234yf, is at least about 50%, more preferably at least about 70% and more preferably at least about 80%.

### EXAMPLES

Additional features of the present invention are provided in the following examples, which should not be construed as limiting the claims in any way.

### Example 1

### Preparation of CH₂=CClCH₂Cl (2,3-Dichloro-1-propene) from CH₂ClCHClCH₂Cl

About 8500 grams of 1,2,3-trichloropropane and about 88.0 grams Aliquat 336 were charged into a 30 liter glass vessel, equipped with TEFLON® shaft and stir blades, heated with internal TEFLON® coated copper coils and refrigerant/heating circulation bath and refrigerated condenser. The mixture was then heated to about 73°C with medium speed agitation. At this temperature, about 10,000 grams of 25 wt% NaOH/H2O solution is added into the reactor from a separate container over a 2 hour period of time. The pH was kept at about 14. After addition, the reaction progress was monitored by GC and GC/MS. The conversion of 1,2,3-trichloropropane was about 97.5% and the selectivity to CH₂=CClCH₂Cl was about 95.4%. After the stipulated reaction time, the mixture was cooled and about 4.0 liters of distilled and ionized water was added into the mixture. The mixture was stirred for about 10 minutes and allowed to separate. The lower layer product (boiling point of about 92.5°C) was drained and distilled to substantially isolate and purify product. The crude yield before distillation was about 6408 grams (GC purity of about 93%).

### Example 2

### Preparation of HCCl₂CCl₂CH₂Cl from CH₂=CClCH₂Cl

Chlorine was bubbled into about 82.4 g of 2,3-dichloropropene at about 10 to about 30 °C with the aid of ice bath cooling until a pale yellow color persisted for about 45 minutes. The crude product in an amount of about 130.4 g, consisted of about 93.6 % CH₂ClCCl₂CH₂Cl and about 2.6 % 2,3-.dichloropropene.

Five hundred grams of CH₂ClCCl₂CH₂Cl was charged into a photoreactor. The jacket for the reactor as well as the jacket for the 450 W UV lamp were cooled to about 15°C using a circulating cooling bath. A total of about 150 g of chlorine was bubbled into the organic liquid over a period of about 2 hours. The crude product weighed about 591 g. GC analysis indicated a conversion of about 54.4 % and selectivity for the desired HCCl₂CCl₂CH₂Cl of about 87 %. Distillation provided HCCl₂CCl₂CH₂Cl in 99 % purity.

### Example 3

### Preparation of CCl₂=CClCH₂Cl from HCCl₂CCl₂CH₂Cl

Aliquat-336® (about 0.26 g) and about 24.8 g of HCCl₂CCl₂CH₂Cl were stirred rapidly at room temperature while adding about 20 g of 25 % aqueous NaOH over 19 minutes. Stirring was continued overnight before adding 30 mL water and allowing the phases to separate. The lower organic phase, in an amount of about 19.8 g, was about 97.5 % pure **CCl₂=CClCH₂Cl**by GC analysis (96 % yield). Prior to fluorination, it was distilled (bp about 69 to about72 °C at about 30 mm Hg) to remove any phase transfer catalyst. H NMR: δ 4.41 (s) ppm.

### Example 4

### Selective catalyzed-transformation of CCl₂=CClCH₂Cl to CF₃CCl=CH₂ (HFO-1233xf) in gas-phase

An 22-inch long and 1/2-inch diameter Monel pipe gas-phase reactor is charged with about 120 cc of a catalyst or a mixture of two catalysts. In case of a mixture, Cr₂O₃ catalyst is kept at the bottom zone of the reactor at a constant temperature of about 270°C-500°C and the other catalyst, such as FeCl₃/C, is kept at the middle and the top zone of the reactor at a constant temperature of about 120°C - 220°C. The reactor is mounted inside a heater with three zones (top, middle, and bottom). The reactor temperature is read by custom-made-5-point thermocouples kept inside at the middle of the reactor. The bottom of the reactor is connected to a pre-heater, which is kept at 300°C by electrical heating. The liquid-HF is fed from a cylinder into the pre-heater through a needle valve, liquid mass-flow meter, and a research control valve at a constant flow of about 1 to about 1000 grams pre hour (g/h). The HF cylinder is kept at a constant pressure of 45 psig by applying anhydrous N₂ gas pressure into the cylinder head space. About 10 to about 1000 g/h of CCl₂=CClCH₂Cl is fed as a liquid through a dip tube from a cylinder under about 45 psig of N₂ pressure. The organic flows from the dip tube to the preheater (kept at about 250°C) through a needle valve, liquid mass-flow meter, and a research control valve at a constant flow of 1-1000 g/h. The organic is also fed as a gas while heating the cylinder containing organic at about 220°C. The gas coming out of the cylinder is passed through a needle valve and a mass flow controller into the preheater. The organic line from the cylinder to the pre-heater is kept at about 200°C by wrapping with constant temperature heat trace and electrical heating elements. All feed cylinders are mounted on scales to monitor their weight by difference. The catalysts are dried at the reaction temperature over a period of about 8 hours and then pretreated with about 50 g/h of HF under atmospheric pressure over a period of about 6 hours and then under 50 psig HF pressure over another period of about 6 hours before contacting with organic feed containing CCl₂=CClCH₂Cl. The reactions are run at a constant reactor pressure of about 0 to about 150 psig by controlling the flow of reactor exit gases by another research control valve. The gases exiting reactor are analyzed by on-line GC and GC/MS connected through a hotbox valve arrangement to prevent condensation. The conversion of CCl₂=CClCH₂Cl is about 70 to about 100% and the selectivity to 1233xf is about 80% to about 95%, respectively. The product is collected by flowing the reactor exit gases through a scrubber solution comprising about 20wt% to about 60 wt% . KOH in water and then trapping the exit gases from the scrubber into a cylinder kept in dry ice or liquid N₂. The product, 1233xf is then substantially isolated by distillation: The results are tabulated in Table 1.

**Table 1: Transformation of CCl₂=CClCH₂Cl to CF₃CCl=CH₂ (CCl₂=CClCH₂Cl + 3HF → CF₃CCl=CH₂ + 3HCl)**

| **#** | **Catalyst** | **T, °C** | **HF flow, g/h** | **CCl₂=CClCH₂Cl flow, g/h** | **% Conv of CCl₂=CClCH₂Cl** | **% Sel to 1233xf** |
|---|---|---|---|---|---|---|
| **1** | 10% v/v Cr₂O₃-90% v/v FeCl₃/C | 350/150 | 50 | 12 | 79 | 81 |
| **2** | 20% v/v Cr₂O₃-80% v/v FeCl₃/C | 350/150 | 50 | 12 | 83 | 86 |
| **3** | 30% v/v Cr₂O₃-70% v/v FeCl₃/C | 350/150 | 50 | 12 | 89 | 96 |
| **4** | 30% v/v Cr₂O₃-70% v/v FeCl₃/C | 350/150 | 70 | 12 | 79 | 93 |
| **5** | 30% v/v Cr₂O₃-70% v/v FeCl₃/C | 345/170 | 50 | 25 | 85 | 90 |
| **6** | Cr₂O₃ | 350 | 50 | 20 | 90 | 93 |
| **7** | FeCl₃/C | 150 | 50 | 20 | 74 | 39 |
| **8** | SbCl₅/C | 150 | 50 | 20 | 81 | 52 |

| | | | | | | |
|---|---|---|---|---|---|---|
| Reaction conditions: Catalyst used (total) 120 cc; pressure, 1.5 psig; | | | | | | |

### Examples 5A and 5B

### Liquid-phase catalytic fluorination of CF₃CCl=CH₂ (1233xf) with HF to CF₃CFClCH₃ (244bb)

### Example 5A

About 327 grams of HF, about 50 grams 1233xf, and about 75 grams SbCl₅ were charged into a 1-L autoclave. The reaction mixture was stirred at a temperature of about 80°C for about 3 hours under about 620 psig of pressure. After the reaction, the reactor was cooled to about 0°C and about 300 ml water was then added slowly into the autoclave over a period of about 45 min. After complete addition of water under stirring, the reactor was cooled to room temperature and then the overhead gases were transferred to another collecting cylinder. The yield of CF₃CFClCH₃ was about 90% at a 1233xf conversion level of about 98%. The other major by-products were CF₃CF₂CH₃ (2%), and an unidentified isomer of a C4 compound of the general formula, C₄H₃Cl₃F₄ (8%).

### Example 5B

About 327 grams HF, about 50 grams 1233xf, and about 75 grams SbCl₅ were charged into a 1-L autoclave. The reaction mixture was stirred at 80°C for about 3 hours under about 625 psig of pressure. After the reaction, the reactor was cooled to about 45°C and then the overhead gas mixture was passed through a well dried KF, NaF, or Al₂O₃ (350g) packed column kept at about 80°C to strip off HF from the gas stream. The gases coming out of the column are collected in a cylinder kept in dry ice (-70°C) bath. The yield of CF₃CFClCH₃ was 87% at a 1233xf conversion level of 93%. The other major by-products were CF₃CF₂CH₃ (1%), and an unidentified isomer of a C4 compound of the general formula, C₄H₃Cl₃F₄ (7%). The product, CF₃CFClCH₃ was isolated by distillation with 98% purity.

### Example 6

### Gas-phase catalytic fluorination of CF₃CCl=CH₂ (1233xf) with HF to CF₃CFClCH₃ (244bb)

A 22-inch (1/2-inch diameter) Monel tube gas phase reactor was charged with about 120 cc of a catalyst. The reactor was mounted inside a heater with three zones (top, middle and bottom). The reactor temperature was read by a custom made 5-point thermocouple kept at the middle inside of the reactor. The inlet of the reactor was connected to a pre-heater, which was kept at about 300°C by electrical heating, Organic (1233xf) was fed from a cylinder kept at 70°C through a regulator, needle valve, and a gas mass-flow-meter. The organic line to the pre-heater was heat traced and kept at a constant temperature of about 73°C by electrical heating to avoid condensation. N₂ was used as a diluent in some cases and fed from a cylinder through a regulator and a mass flow controller into the pre-heater. All feed cylinders were mounted on scales to monitor their weight by difference. The reactions were run at a constant reactor pressure of from about 0 to about 100 psig by controlling the flow of reactor exit gases by another research control valve. The gas mixtures exiting reactor was analyzed by on-line GC and GC/MS connected through a hotbox valve arrangements to prevent condensation. The conversion of 1233xf was from about 50% to about 65% and the selectivity to 244 isomer (CF₃CFClCH₃) was from about 90% to about 93% depending on the reaction conditions using 120 cc of 50 wt% SbCl₅/C as the catalyst at about 65°C to about -85°C with a HF flow of about 50 g/h and organic flow of about 15 g/h. No CF₃CF₂CH₃ was observed under the reaction conditions. The catalyst is pretreated at first with 50 g/h HF at about 65°C for about 2 hours and then with about 50 g/h HF and about 200 seem of Cl₂ at about 65°C for about 4 hours. After pre-treatment, about 50 seem of N₂ is flows over a period of about 40 minutes through the catalyst bed to sweep free chlorine from the catalyst surface prior to interacting with the organic feed (1233xf). Pretreatment is considered important to many embodiments of the invention. The products were collected by flowing the reactor exit gases through a 20-60 wt% aqueous KOH scrubber solution and then trapping the exit gases from the scrubber into a cylinder kept in dry ice or liquid N₂. The products were then isolated by distillation. About 50 wt% SbCl₅/C, about 3 to about 6 wt% FeCl₃/C, , 20 wt% SnCl₄/C, and about 23 wt% TiCl₄/C, using 4 different kind of activated carbon such as Shiro saga, Calgon, Norit, and Aldrich were used as the catalyst at from about 60 to about 150°C. Among all the catalysts used for this reaction, Cl₂ and HF pre-treated SbCl₅/C was found to be generally preferred in terms of activity. The results using SbCl₅ as the catalyst are shown in Table 2.

**Table 2: Catalyzed-gas-phase transformation of CF₃CCl=CH₂to CF₃CFClCH₃**

| # | Cat | T, °C | Conv. of CF₃CCl=CH₂ (1233xf) | Sel. to CF₃CFClCH₃ |
|---|---|---|---|---|
| 1 | 10 wt% SbCl₅/C | 60 | 15 | 100 |
| 2 | 20 wt% SbCl₅/C | 60 | 21 | 98 |
| 3 | 30 wt% SbCl₅/C | 60 | 32 | 98 |
| 4 | 50 wt% SbCl₅/C | 60 | 55 | 97 |
| 5 | 50 wt% SbCl₅/C | 80 | 62 | 93 |
| 6 | 50 wt% SbCl₅/C | 100 | 56 | 87 |
| 7 | 60 wt% SbCl₅/C | 60 | 59 | 91 |
| 8 | 50 wt% SbCl₅/NORIT RFC 3 Activated Carbon | 60 | 34 | 92 |
| 9 | 50 wt% SbCl₅/Shiro Saga Activated Carbon | 60 | 56 | 96 |
| 10 | 50 wt% SbCl₅/Aldrich Activated Carbon | 60 | 57 | 94 |

**Reaction conditions:** 1233xf flow, 150 sccm; HF flow 50 g/h; pressure, 2.5-5.3 psig; in 1-5 reactions Calgon activated carbon is used as the catalyst support; catalyst, 120 cc. All catalysts are pre-treated with Cl₂ and HF prior to contacting with 1233xf.

### Example 7

### Conversion of CF₃CFClCH₃ to CF₃CF=CH₂ in gas-phase

A 22-inch (1/2-inch diameter) Monel tube gas phase reactor was charged with 120 cc of catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic (CF₃CFClCH₃) was fed from a cylinder kept at about 65°C through a regulator, needle valve, and a gas mass-flow-meter. The organic line to the pre-heater was heat traced and kept at a constant temperature of from about 65°C to about 70°C by electrical heating to avoid condensation. The feed cylinder was mounted on scales to monitor their weight by difference. The reactions were run at a constant reactor pressure of from about 0 to about 100 psig by controlling the flow of reactor exit gases by another research control valve. The gas mixture exiting reactor was analyzed by on-line GC and GC/MS connected through a hotbox valve arrangement to prevent condensation. The conversion of CF₃CFClCH₃ was almost 98% and the selectivity to HFO-1234yf was from about 69% to about 86% depending on the reaction conditions. The products were collected by flowing the reactor exit gases through a about 20wt% to about 60 wt% of aquesous KOH scrubber solution and then trapping the exit gases from the scrubber into a cylinder kept in dry ice or liquid N₂. The products were then isolated by distillation. Results are tabulated in T-able 3.

**Table 3: Catalyzed-transformation of CF₃CFClCH₃ to HFO-1234yf**

| # | Cat | T, °C | | Flow rate, CF₃CFClCH₃ (244 isomer) seem | Conversion of 244 | 1234yf (Sel. %) |
|---|---|---|---|---|---|---|
| 1 | A | 400 | | 150 | 100 | 46 |
| 2 | B | 400 | | 150 | 96 | 63 |
| 3 | C | 400 | | 100 | 100 | 64 |
| 4 | D | 400 | | 100 | 99 | 93 |
| 5 | D | 400 | | 150 | 92 | 89 |
| 6 | E | 400 | | 100 | 96 | 56 |
| 7 | F | 400 | | 100 | 87 | 51 |
| 8 | G | 400 | | 100 | 100 | 37 |

**Reaction conditions**: pressure, 2.5-5.3 psig; catalyst, 100 cc, A is NORIT RFC 3; B is Shiro-Saga activated carbon; C is Aldrich activated carbon; D is Calgon activated carbon; activated carbon; E is 0.5 wt% Pd/C; F is 0.5 wt% Pt/C; G is Ni-mesh; Organic cylinder temperature-65°C; CF₃CFClCH₃ (244) line to the preheater-60°C; Preheater, 350°C; P-5 psig.

### Example 8

### Selective catalyzed-transformation of CCl₃CCl=CH₂ to CF₃CCl=CH₂ (HFO-1233xf) in gas-phase

A 22-inch long and 1/2-inch diameter Monel pipe gas phase reactor was charged with 120 cc of a catalyst or a mixture of two catalysts. In case of a mixture, Cr₂O₃ catalyst is kept at the bottom zone of the reactor at a substantially constant temperature of from about 270°C to about 500°C and the other catalyst, such as FeCl₃/C is kept at the middle and the top zone of the reactor at a substantially constant temperature of from about 120°C to about 220°C. The reactor was mounted inside a heater with three zones (top, middle, and bottom). The reactor temperature was read by custom-made-5-point thermocouples kept inside at the middle of the reactor. The bottom of the reactor was connected to a pre-heater, which was kept at about 300°C by electrical heating. The liquid-HF was fed from a cylinder into the pre-heater through a needle valve, liquid mass-flow meter, and a research control valve at a substantially constant flow of from about 1 to about 1000 g/h. The HF cylinder was kept at a substantially constant pressure of about 45 psig by applying anhydrous N₂ gas pressure into the cylinder head space. A feed rate of from about 10 g/h to about 1000 g/h of CCl₃CCl=CH₂ was fed as a liquid through a dip tube from a cylinder under about 45 psig of N₂ pressure. The organic was flown from the dip tube to the pre-heater (kept at about 250°C) through needle valve, liquid mass-flow meter, and a research control valve at a substantially constant flow of from about 1 to about 1000 g/h. The organic is also fed as a gas while heating the cylinder containing organic at about 220°C. The gas effluent from the cylinder is passed through a needle valve and a mass flow controller into the pre-heater. The organic line from the cylinder to the pre-heater was kept at about 200°C by wrapping with constant temperature heat trace and electrical heating elements. All feed cylinders were mounted on scales to monitor their weight by difference. The catalysts were dried at the reaction temperature over a period of about 8 hours and then pretreated with about 50 g/h of HF under atmospheric pressure over a 6 hour period and then under about 50 psig HF pressure over a 6 hour period before contacting with organic feed, CCl₃CCl=CH₂. The reactions were run at a substantially constant reactor pressure ranging from about 0 to about 150 psig by controlling the flow of reactor exit gases by another research control valve. Those gases exiting reactor were analyzed by on-line GC and GC/MS connected through a hotbox valve arrangements to prevent condensation. The conversion of GCl₃CCl=CH₂ was in a range of from about 90% to about 100% and the selectivity to CF₃CCl=CH₂ (1233xf) was about 79%.. The effluent contained in addition HFO-1243zf in an amount of about 7.7%, 1232-isomer in an amount of about 1.3%, and 1223 in an amount of about 0.8%, and an unidentified byproduct. The product was collected by flowing the reactor exit gases through a 20-60 wt% aq. KOH scrubber solution and then trapping the exit gases from the scrubber into a cylinder kept in dry ice or liquid N₂. The product, 1233xf was then substantially isolated by distillation. Using only Cr₂O₃ catalyst, a selectivity of about 68% to 1233xf at a conversion level of about 79% was achieved.

### Examples 9A - 9D

### Direct Liquid-phase catalytic fluorination of CCl₃CCl=CH₂ with HF to CF₃CFClCH₃ (244-isomer)

### Example 9A

About 327 grams HF, about 50 grams CCl₃CCl=CH₂, and about 75 grams SbCl₅ were charged into a 1-L autoclave. The reaction mixture was stirred at about 80°C for about 3 hours under about 610 psig of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The yield of CF₃CFClCH₃ was about 89% at a CCl₃CCl=CH₂ conversion level of about 88%. The other major by-products were CF₃CF₂CH₃ (2%), and an unidentified isomer of a C4 compound of the general formula, C₄H₃Cl₃F₄ (8%).

### Example 9B

About 327 grams HF, about 50 grams CCl₃CCl=CH₂, and about 75 grams SbCl₅ were charged into a 1-L autoclave. The reaction mixture was stirred at about 100°C for about 3 hours under about 685 psig of pressure. After the reaction, the reactor was cooled to about 40°C and about 300 ml water was then added slowly into the autoclave over a period of about 45 minutes. After complete addition of water. under stirring, the reactor was cooled to room temperature and then the overhead gases were transferred to another collecting cylinder. The yield of CF₃CFClCH₃ was about 78% at a CCl₃CCl=CH₂ conversion level of about 100%. The other major by-products were CF₃CF₂CH₃ (about 4%), and an unidentified isomer of a C4 compound of the general formula, C₄H₃Cl₃F₄ (about 13%).

### Example 9C

About 327 grams HF, about 50 grams CCl₃CCl=CH₂, and about 75 grams SbCl5 were charged into a 1-L autoclave. The reaction mixture was stirred at about 125°C for about 6 hours under about 825 psig of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The major products were CF₃CF₂CH₃ (about 53%) and CF₃CFClCH₃ (about 25%) at a CCl₃CCl=CH₂ conversion level of about 100%. The other major by-products were and unidentified isomer of a C4 compound of the general formula, C₄H₃Cl₃F₄ (8%) and tar.

### Example 9D

About 327 grams HF, about 50 grams CCl₃CCl=CH₂, and about 75 g SbCl₅ were charged into a 1-L autoclave. The reaction mixture was stirred at about 150°C for about 6 hours under about 825 psig of pressure. After the reaction, the reactor was cooled to about 40°C and about 300 ml water was then added slowly into the autoclave over a period of about 45 minutes. After complete addition of water under stirring, the reactor was cooled to about room temperature and then the overhead gases were transferred to another collecting cylinder. The major products were CF₃CF₂CH₃ (about 57%) and CF₃CFClCH₃ (about 15%) at a CCl₃CCl=CH₂ conversion level of about 100%. The other major by-products were and unidentified isomer of a C4 compound of the general formula, C₄H₃Cl₃F₄ (about 11%) and tar.

### Example 10

### Catalytic conversion of CF₃CF₂CH₃ to CF₃CF=CH₂

A 22-inch (1/2-inch diameter) Monel tube gas phase reactor was charged with 120 cc of a catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic material (245cb) was fed from a cylinder kept at about 65°C through a regulator, needle valve, and a gas mass-flow-meter. The organic line to the pre-heater was heat traced and kept at a substantially constant temperature in a range of from about 65°C to about 70°C by electrical heating to avoid condensation. The feed cylinder was mounted on a scale to monitor its weight by difference. The reactions were run at a substantially constant reactor pressure of from about 0 to about 100 psig by controlling the flow of reactor exit gases by another research control valve. The gas mixtures exiting reactor was analyzed by on-line GC and GC/MS connected through a hotbox valve arrangements to prevent condensation. The conversion of 245cb was in the range of from about 30% to about 70% and the selectivity to 1234yf was in the range of from about 90% 50 about 100% depending on the reaction conditions. The products were collected by flowing the reactor exit gases through a 20-60-wt% of aq. KOH scrubber solution and then trapping the exit gases from the scrubber into a cylinder kept in dry ice or liquid N₂. The products were then substantially isolated by distillation. Results are tabulated in Table 4.

**Table 4: Transformation of CF₃CF₂CH₃ to 1234yf**

| # | Cat | T, °C | H₂, sccm | CF₃CF₂CH₃ (245cb) sccm | Conversion of 245cb, % | 1234yf (sel. %) |
|---|---|---|---|---|---|---|
| 1 | A | 575 | 0 | 65 | 79 | 63 |
| 2 | B | 575 | 0 | 68 | 82 | 57 |
| 3 | C | 575 | 0 | 73 | 73 | 61 |
| 4 | D | 575 | 0 | 68 | 84 | 59 |
| 5 | D | 575 | 20 | 68 | 89 | 73 |
| 6 | E | 550 | 0 | 69 | 92 | 53 |
| 7 | F | 550 | 0 | 67 | 93 | 33 |
| 8 | G | 550 | 0 | 69 | 73 | 46 |

**Reaction conditions:** pressure, 2.5-5.3 psig; catalyst, 100 cc, A is NORIT RFC 3; B is Shiro-Saga activated carbon; C is Aldrich activated carbon; D is Calgon activated carbon; E is 0.5 wt% Pd/C; F is 0.5 wt% Pt/C; G is Ni-mesh; Organic cylinder temperature is about 65°C; CF₃CF₂CH₃ (245cb) line to the preheater is maintained at about 50°C; preheater temperature is maintained at about 350°C; N₂ flow is not used; pressure is maintained at about 3 psig.

Having thus described a few particular embodiments of the invention, various alterations, modifications, and improvements will readily occur to those skilled in the art. Such alterations, modifications, and improvements, as are made obvious by this disclosure, are intended to be part of this description though not expressly stated herein, and are intended to be within the spirit and scope of the invention. Accordingly, the foregoing description is by way of example only, and not limiting. The subject matter encompassed by the following numbered embodiments also forms part of the present invention, optionally in combination with the subject matter described above and/or defined in any claims that follow.

### Numbered Embodiments

### Numbered Embodiment 1

A method for producing fluorinated organic compounds comprising converting at least one compound of Formula (I):

C(X)ₘCCl(Y)ₙC(X)ₘ (I)

to at least one compound of Formula (II)

CF₃CF=CHZ (II)

where X, Y and Z is independently H, F, Cl, I or Br, and each m is independently 1, 2 or 3, and n is 0 or 1.

### Numbered Embodiment 2

The method of Numbered Embodiment 1 wherein said at least one compound of Formula (I) comprises a compound where n is 0, each X is independently H or Cl, and Z is H.

### Numbered Embodiment 3

The method of Numbered Embodiment 1 wherein said at least one compound of Formula (I) comprises a compound Formula (IA):

C(X)₂=CClC(X)₃ (IA)

wherein X is as identified in Numbered Embodiment 1.

### Numbered Embodiment 4

The method of Numbered Embodiment 3 wherein Z in said compound of Formula (II) is H.

### Numbered Embodiment 5

The method of Numbered Embodiment 3 wherein X is independently H or Cl.

### Numbered Embodiment 6

The method of Numbered Embodiment 1 wherein said at least one compound of Formula (I) comprises at least one tetrachloropropene.

### Numbered Embodiment 7

The method of Numbered Embodiment 1 wherein said at least one compound of Formula (I) is selected from the group consisting of CH₂=CClCCl₃, CCl₂=CClCH₂Cl, CHCl=CClCCl₂H, and combinations of these.

### Numbered Embodiment 8

The method of Numbered Embodiment 1 wherein said at least one compound of Formula (I) comprises a compound Formula (IAA):

C(X)₂=CClCF₃ (IAA)

wherein X is as identified in Numbered Embodiment 1.

### Numbered Embodiment 9

The method of Numbered Embodiment 8 wherein each X in said compound of Formula (IAA) is independently H or Cl.

### Numbered Embodiment 10

The method of Numbered Embodiment 1 wherein said at least one compound of Formula (I) comprises CH₂=CClCF₃ (HCFC-1223xf).

### Numbered Embodiment 11

The method of Numbered Embodiment 1 wherein said at least one compound of Formula (I) comprises a compound of Formula (IB):

C(X)₃CClYC(X)₃ (IB)

### Numbered Embodiment 12

The method of Numbered Embodiment 11 wherein said Formula (IB) compound has at least two halogens on one terminal carbon and at least two hydrogen atoms on the other terminal carbon.

### Numbered Embodiment 13

The method of Numbered Embodiment 12 wherein said at least one compound of Formula (IB) comprises at least one propene having at least four halogens substituents.

### Numbered Embodiment 14

The method of Numbered Embodiment 12 wherein said at least one compound of Formula (IB) comprises at least one propene having at least five halogen substituents.

### Numbered Embodiment 15

The method of Numbered Embodiment 12 wherein Y is F and all three X on one terminal carbon are F in said at least one compound of Formula (IB).

### Numbered Embodiment 16

The method of Numbered Embodiment 12 wherein said at least one compound of Formula (IB) is selected from the group consisting of 1,1,1,2-tetrafluoro-2-chloropropane, 1-chloro-1,3,3,3-tetrafluoropropane (HFC-244fa), CH₂ClCHClCCl₃, CHCl₂CCl₂CH₂Cl, CHCl₂CHClCHCl₂, and combinations of these.

### Numbered Embodiment 17

The method of Numbered Embodiment 1 wherein said compound of Formula (I) comprises at least one compound of Formula (IA):

C(X)₂=CClC(X)₃ (IA)

and said converting step comprises converting said Formula (IA) compound to a compound of Formula (IAA)

C(X)₂=CClCF₃ (IAA)

and then converting said compound of Formula (IAA) to a compound of Formula (IB)

C(X)₃CClYC(X)₃ (IB)

wherein X and Y are each as identified in Numbered Embodiment 1, and then converting said compound of Formula (IB) to a compound of Formula (II).

### Numbered Embodiment 18

The method of Numbered Embodiment 17 wherein said compound of Formula (IAA) comprises CF₃CCl=CH₂ (HFO-1223xf) under conditions effective to produce at least one monochlorotetrafluoropropane in accordance with Formula (IB).

### Numbered Embodiment 19

The method of Numbered Embodiment 17 wherein said monochlorotetrafluoropropane in accordance with Formula (IB) comprises CF₃CFClCH₃ (HFC-244fa).

### Numbered Embodiment 20

The method of Numbered Embodiment 19 wherein said CF₃CFClCH₃ (HFC-244fa) is exposed to reaction conditions effective to produce at least one compound in accordance with Formula (II) wherein Z is H.

### Numbered Embodiment 21

The method of Numbered Embodiment 20 wherein said exposing step comprises at least one gas phase catalytic reaction.

### Numbered Embodiment 22

The method of Numbered Embodiment 1 wherein said exposing step comprises reacting said at least one compound of Formula (I) in which n is 0 under conditions effective to produce at least one chlorofluoropropane.

### Numbered Embodiment 23

The method of Numbered Embodiment 22 wherein said at least one chlorofluoropropenes is a compound in accordance with Formula (IBB):

CF₃CClFC(X)₃ (IBB)

where each X is independently F, Cl or H.

### Numbered Embodiment 24

The method of Numbered Embodiment 23 wherein at least one of said X in Formula (IBB) is H.

### Numbered Embodiment 25

The method of Numbered Embodiment 24 wherein all three X in Formula (IBB) are H.

### Numbered Embodiment 26

A method for producing fluorinated organic compounds comprising providing at least one compound of Formula (I)

C(X)ₘCCl(Y)ₙC(X)ₘ (I)

and exposing said Formula (I) compound to reaction conditions effective to convert at least about 50% of said compound to a compound of Formula (II)

CF₃CF=CHZ (II)

where each X, Y and Z is independently H, F, Cl, I or Br, and each m is independently 1, 2 or 3, and n is 0 or 1.

### Numbered Embodiment 27

The method of Numbered Embodiment 26 wherein said exposing step comprises exposing said Formula (I) compound to reaction conditions effective to convert at least about 90% of said compound to a compound of Formula (II).

## Claims

1. A method for the catalytic conversion of CF₃CH₂CH₃ to CF₃CF=CH₂.

2. The method of claim 1 wherein said method comprises:
charging a reaction with a catalyst;
adding CF₃CH₂CH₃ at 65°C;
converting CF₃CH₂CH₃ to CF₃CF=CH₂ at a constant reactor pressure of 0-100psig;
collecting the products by flowing the reactor exit gases through a 20-60 wt% of aq KOH scrubber solution, trapping the gasses in a cylinder kept in dry ice or liquid N₂ and substantially isolating the products by distillation;
wherein the catalyst is selected from NORIT RFC 3, activated carbon, 0.5 wt% Pd/C, or Ni-mesh;
wherein the conversion of CF₃CH₂CH₃ is 30% to 70% and the selectivity of CF₃CF=CH₂ is 90% to 100%.
